# EUROPÄISCHE PATENTSCHRIFT

(11) **EP 3 706 821 B1**
(45) Veröffentlichungstag und Bekanntmachung des Hinweises auf die Patenterteilung: **30.08.2023**
(21) Anmeldenummer: 18807562.6
(22) Anmeldetag: 08.11.2018
(51) Int. Cl.: A61M 1/16, B01D 19/00

(54) **VERFAHREN UND VORRICHTUNG ZUR ENTGASUNG VON FLÜSSIGKEITEN**
METHOD AND DEVICE FOR DEGASSING LIQUIDS
PROCÉDÉ ET DISPOSITIF POUR DÉGAZER DES LIQUIDES

(30) Priorität: 08.11.2017 DE 102017126136
(43) Veröffentlichungstag der Anmeldung: 16.09.2020
(73) Patentinhaber: Fresenius Medical Care Deutschland GmbH, 61352 Bad Homburg (DE)
(72) Erfinder: PETERS, Arne, 61352 Bad Homburg (DE); FISCHER, Gerome, 99947 Weberstedt (DE)
(74) Vertreter: Herrmann, Uwe
(86) Internationale Anmeldenummer: PCT/EP2018/080605
(87) Internationale Veröffentlichungsnummer: WO 2019/092101

(56) Entgegenhaltungen:
- WO-A1-2017/137178
- WO-A1-2017/137178
- DE-A1-102015 119 237
- GB-A- 561 366
- US-A- 4 371 385
- US-A- 4 371 385

## Beschreibung

Die vorliegende Erfindung betrifft ein Verfahren und eine Vorrichtung zur Entgasung von Flüssigkeiten sowie die Verwendung der erfindungsgemäß bereitgestellten Flüssigkeit.

Aus dem Stand der Technik ist es bekannt, Flüssigkeiten zur Herstellung medizinscher Lösungen und insbesondere von Dialyselösungen zu verwenden, wobei zur Förderung dieser Flüssigkeiten häufig nicht okkludierende Pumpen, wie z.B. Kreiselpumpen oder Impellerpumpen verwendet werden. Diese Pumpen haben zahlreiche Vorteile. Sie sind allerdings mit dem Nachteil behaftet, dass sich Luftblasen im Innenraum der Pumpen ansammeln können, wodurch die Förderleistung der Pumpen verringert wird und ggf. Schäden an der Pumpe, z.B. durch Kavitation hervorgerufen werden können, was die Lebensdauer der Pumpe verringert.

Dokument WO2017/137178 offenbart eine Vorrichtung zur extrakorporalen Blutbehandlung und Verfahren zum Betreiben einer Vorrichtung zur extralorporalen Blutbehandlung.

Der vorliegenden Erfindung liegt daher die Aufgabe zugrunde, eine Vorrichtung und ein Verfahren zur Herstellung einer Flüssigkeit bereitzustellen, bei der/dem dieses Problem nicht oder nur in untergeordnetem Umfang auftritt.

Diese Aufgabe wird durch eine Vorrichtung mit den Merkmalen des Anspruchs 1 sowie durch ein Verfahren mit den Merkmalen des Anspruchs 9 gelöst.

Vorzugsweise werden zwei Luftabscheidekammern, im Folgenden auch als Entgasungskammern oder Kammern bezeichnet, verwendet, wobei sich vorzugsweise eine der Kammern hydraulisch vor und vorzugsweise die andere hydraulisch hinter der Pumpe befindet.

Vorzugsweise verfügt jede der Entgasungskammern über je ein Ventil zum atmosphärischen Druckausgleich.

Die Entgasungskammern sind durch eine Rückführungsleitung miteinander verbunden, so dass Flüssigkeit von der zweiten zu der ersten Entgasungskammer strömen kann. Diese Rückführungsleitung weist vorzugsweise ein Absperrmittel, z.B. ein Ventil, eine Schlauchklemme oder dergleichen auf, mittels derer diese absperrbar ist und geöffnet werden kann.

Die Vorrichtung zur Entgasung von Flüssigkeiten gemäß der Erfindung umfasst ein Entgasungssystem, wobei das Entgasungssystem eine erste Entgasungskammer, eine zweite Entgasungskammer, einen Flüssigkeitsvorrat, eine Pumpe sowie eine Zufuhrleitung aufweist, die den Flüssigkeitsvorrat mit der ersten Entgasungskammer verbindet, wobei die Pumpe saugseitig mit der ersten Entgasungskammer und druckseitig mit der zweiten Entgasungskammer verbunden ist, wobei das Entgasungssystem des Weiteren eine absperrbare Rückführungsleitung aufweist, die die beiden Entgasungskammern miteinander verbindet. Die Vorrichtung weist darüber hinaus einen Kontroller, wie z.B. eine Steuer- oder Regelungseinheit, auf, der ausgebildet ist, das Entgasungssystem in einem ersten Betriebsmodus und in einem zweiten Betriebsmodus zu betreiben, wobei in dem ersten Betriebsmodus das Entgasungssystem derart geschaltet ist, dass die Pumpe Flüssigkeit aus der ersten Entgasungskammer abführt und einer Abnahmeeinheit für entgaste Flüssigkeit zuführt, und wobei das Entgasungssystem in dem zweiten Betriebsmodus derart geschaltet ist, dass Flüssigkeit durch die Rückführungsleitung aus der zweiten in die erste Entgasungskammer rückgeführt wird.

Der erste Betriebsmodus ist somit dadurch gekennzeichnet, dass die Pumpe, vorzugsweise eine nicht okkludierende Pumpe, unmittelbar oder mittelbar aus der ersten Entgasungskammer Flüssigkeit ansaugt, die zumindest teilweise entgast ist, und diese Flüssigkeit unmittelbar oder mittelbar zu einer Abnahmeeinheit fördert. Bei dieser Abnahmeeinheit kann es sich beispielsweise um einen Verteiler oder um eine Leitung, einen Behälter etc. oder auch um eine Vorrichtung handeln, mittels derer eine medizinische Flüssigkeit, insbesondere eine Dialyselösung hergestellt werden kann. Bei der Vorrichtung kann es sich auch um ein Dialysegerät handeln, das Mittel zur Herstellung einer Dialyselösung aus der bereitgestellten Flüssigkeit aufweist.

Die Entgasung findet in der ersten Entgasungskammer und ggf. auch schon vor Eintritt der Flüssigkeit in die erste Entgasungskammer, nämlich in der Zufuhrleitung statt, durch die Flüssigkeit von dem Flüssigkeitsvorrat in die erste Entgasungskammer strömt bzw. durch die Pumpe gesaugt wird. Dazu kann in der Zufuhrleitung eine Drossel angeordnet sein, die einen Druckabfall bewirkt, der zu einer verbesserten Entgasung der Flüssigkeit führt.

Bei der Flüssigkeit, die durch die erfindungsgemäße Vorrichtung oder das erfindungsgemäße Verfahren hergestellt wird, handelt es sich vorzugsweise um Wasser, wie z.B. RO-Wasser.

Die Pumpe kann saugseitig unmittelbar oder mittelbar mit der ersten Entgasungskammer und druckseitig unmittelbar oder mittelbar mit der zweiten Entgasungskammer verbunden ist. Denkbar ist beispielsweise, dass die Pumpe saugseitig in dem ersten Betriebsmodus direkt aus der ersten Entgasungskammer oder indirekt, wie z.B. von der ersten über die zweite Entgasungskammer Flüssigkeit ansaugt.

Druckseitig kann vorgesehen sein, dass die Pumpe unmittelbar zu der Abnahmeeinheit fördert oder auch mittelbar, beispielsweise dergestalt, dass die Pumpe zunächst in die zweite Entgasungskammer fördert und die Flüssigkeit von dieser zu der Abnahmeeinheit gelangt.

In einer weiteren Ausgestaltung der Erfindung ist vorgesehen, dass der Kontroller derart ausgebildet ist, dass die Pumpe in beiden Betriebsmodi in einem Fördermodus betrieben wird oder dass die Pumpe nur in dem ersten Betriebsmodus, nicht aber in dem zweiten Betriebsmodus in einem Fördermodus betrieben wird. Unter "Fördermodus" wird verstanden, dass die Pumpe eine Nettoförderung erzeugt, mit der Flüssigkeit zu der Abnahmeeinheit gefördert wird, also keine Kreislaufströmung, bei die Pumpe zwar ebenfalls in Betrieb ist, aber keine Flüssigkeit zu der Abnahmeeinheit gefördert wird.

Denkbar ist es, dass die erste und die zweite Entgasungskammer derart relativ zueinander angeordnet sind, dass in dem zweiten Betriebsmodus die Flüssigkeit durch die Rückführungsleitung aus der zweiten in die erste Entgasungskammer schwerkraftbedingt gelangt oder dass dies bedingt durch die Förderkraft der Pumpe erfolgt.

In einer weiteren Ausführungsform können die beiden Entgasungskammern räumlich voneinander getrennt angeordnet sein.

Von der Erfindung ist jedoch auch der Fall umfasst, dass diese in einer gemeinsamen baulichen Einheit angeordnet sind, die eine Trennwand aufweist, die die beiden Entgasungskammern voneinander trennt. Die Trennwand kann durch ein Ventil oder durch eine sonstige Absperreinheit geöffnet und geschlossen werden.

In einer weiteren Ausgestaltung der Erfindung ist nur die erste Entgasungskammer mit dem Flüssigkeitsvorrat verbunden. In diesem Fall besteht keine Fluidverbindung zwischen der zweiten Entgasungskammer und dem Flüssigkeitsvorrat.

Von der Erfindung ist jedoch auch der Fall umfasst, dass beide Entgasungskammern mit dem Flüssigkeitsvorrat verbunden bzw. verbindbar sind, so dass je nach dem durch den Kontroller vorgegeben Betriebsmodus die erste oder die zweite Entgasungskammer mit dem Flüssigkeitsvorrat in Fluidverbindung steht.

Um eine weitgehend oder vollständig kontinuierliche Bereitstellung entgaster Flüssigkeit an der Abnahmeeinheit zu ermöglichen, können zwei parallel geschaltete Entgasungssysteme vorgesehen sein, wobei der Kontroller derart ausgebildet ist, dass die Entgasungssysteme abwechselnd derart betrieben werden, dass das erste Entgasungssystem in dem ersten Betriebsmodus und das zweite Entgasungssystem zeitgleich in dem zweiten Betriebsmodus betrieben wird und umgekehrt. Die beiden Entgasungssysteme können identisch oder auch unterschiedlich ausgebildet sein.

Vorzugsweise weisen beide der Entgasungskammern wenigstens einen Levelsensor auf, mittels dessen der Füllstand in der Entgasungskammer gemessen oder jedenfalls das Über- oder Unterschreiten eines Grenzwertes des Füllstandes erfasst werden kann.

Der Kontroller kann ausgebildet sein, von dem ersten auf den zweiten Betriebsmodus oder von dem zweiten auf den ersten Betriebsmodus umzuschalten, wenn ein Levelsensor auslöst. Vorzugsweise ist der Kontroller derart ausgeführt, dass dieser von dem ersten auf den zweiten Betriebsmodus umschaltet, wenn der Levelsensor der ersten Entgasungskammer einen Grenzwert unterschreitet und/oder der Levelsensor in der zweiten Entgasungskammer einen Grenzwert übersteigt, und/oder von dem zweiten auf den ersten Betriebsmodus umschaltet, wenn der Levelsensor in der ersten Entgasungskammer einen Grenzwert übersteigt und/oder in der zweiten Entgasungskammer einen Grenzwert unterschreitet.

Die vorliegende Erfindung betrifft des Weiteren ein Verfahren mit den Merkmalen des Anspruchs 9.

Danach ist vorgesehen, dass in dem ersten Betriebsmodus Flüssigkeit aus der ersten Entgasungskammer abgezogen und an einer Abnahmeeinheit für entgaste Flüssigkeit zur Verfügung gestellt wird und dass in dem zweiten Betriebsmodus Flüssigkeit von der zweiten Entgasungskammer in die erste Entgasungskammer zurückgeführt wird.

Dabei kann in dem ersten Betriebsmodus Flüssigkeit aus der ersten Entgasungskammer über die zweite Entgasungskammer mittels der Pumpe zu einer Abnahmeeinheit gefördert werden oder Flüssigkeit direkt aus der ersten Entgasungskammer (ohne Einbeziehung der zweiten Entgasungskammer) zu einer Abnahmeeinheit bzw. zu der zweiten Kammer gefördert werden.

Vorzugsweise wird von dem ersten auf den zweiten Betriebsmodus umgeschaltet, wenn das Flüssigkeitsniveau in der ersten Entgasungskammer einen Grenzwert unterschreitet und/oder in der zweiten Entgasungskammer einen Grenzwert überschreitet.

Vorzugsweise wird von dem zweiten auf den ersten Betriebsmodus umgeschaltet, wenn das Flüssigkeitsniveau in der zweiten Entgasungskammer einen Grenzwert unterschreitet und/oder in der ersten Entgasungskammer einen Grenzwert überschreitet.

Denkbar ist es, dass entgaste Flüssigkeit nur in dem ersten Betriebsmodus oder sowohl in dem ersten sowie auch in dem zweiten Betriebsmodus an der Abnahmeeinheit bereitgestellt wird.

In einer weiteren Ausgestaltung der Erfindung ist vorgesehen, dass beide Entgasungskammern in einer gemeinsamen baulichen Einheit angeordnet sind, die eine Trennwand aufweist, die die beiden Entgasungskammern voneinander trennt, wobei in der Trennwand ein Ventil oder ein sonstiges Absperrelement vorgesehen ist, das in dem ersten Betriebsmodus geöffnet und in dem zweiten Betriebsmodus geschlossen ist.

Die vorliegende Erfindung betrifft des Weiteren die Verwendung einer Vorrichtung gemäß einem der Ansprüche 1 bis 8 oder eines Verfahrens gemäß einem der Ansprüche 9 bis 14 zur Herstellung einer medizinischen Lösung, insbesondere zur Herstellung einer Dialyselösung.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile der Erfindung werden anhand eines in der Zeichnung dargestellten Ausführungsbeispiels näher erläutert.

Es zeigen:
- Figur 1 - 4:: eine schematische Darstellung der Vorrichtung gemäß der Erfindung in einer ersten Ausführungsform in unterschiedlichen Betriebsmodi,
- Figur 5-8:: eine schematische Darstellung der Vorrichtung gemäß der Erfindung in einer zweiten Ausführungsform in unterschiedlichen Betriebsmodi,
- Figur 9 - 14:: eine schematische Darstellung der Vorrichtung gemäß der Erfindung in einer dritten Ausführungsform in unterschiedlichen Betriebsmodi und
- Figur 15:: eine vereinfachte schematische Darstellung der Vorrichtung gemäß der Erfindung.

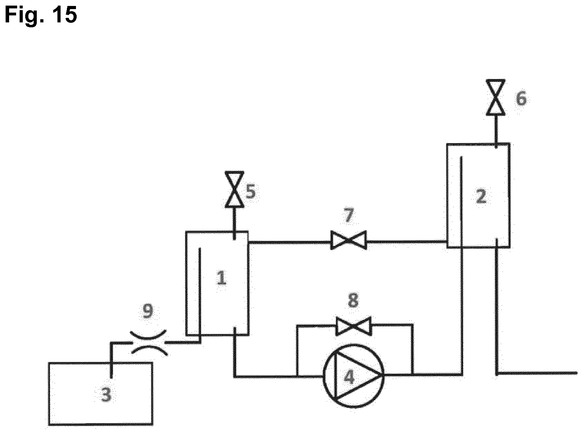

Figur 15 zeigt eine vereinfachte Darstellung der Vorrichtung gemäß der Erfindung.

Die Vorrichtung umfasst zwei Entgasungs- bzw. Luftabscheidekammern 1, 2, wobei sich die eine der Kammern hydraulisch vor und eine der Kammern hydraulisch hinter der Pumpe 4 befindet.

In den Figuren ist ein Nachfüllanschluss zur Nachfüllung des Vorratsbehälters 3 nicht dargestellt.

Die Luftabscheidekammern werden im Rahmen der vorliegenden Erfindung stellvertretend auch als Entgasungskammern oder einfach als Kammern bezeichnet.

Beide Kammern 1, 2 besitzen oben jeweils ein Ventil 5, 6 zum atmosphärischen Druckausgleich und sind durch eine Rückführungsleitung mit einem darin befindlichen Ventil 7 miteinander verbunden. Der Begriff "Ventil" steht im Rahmen der vorliegenden Erfindung stellvertretend für ein beliebiges Absperrorgan. Dabei kann es sich z.B. um eine Schlauchklemme, ein Ventil oder dgl. handeln.

Die Luftabscheidung aus der Flüssigkeit wird wie folgt durchgeführt:
Flüssigkeit wird aus dem Vorratsbehälter 3 oder über eine Zuleitung optional über eine Drossel 9 entgast. Das Luft-Wassergemisch gelangt in die Luftabscheidekammer 1. Hieraus fördert die nicht okkludierende Pumpe 4 und fördert entgaste Flüssigkeit in die zweite Luftabscheidekammer 2. Die Kammer 2 kann bei geöffnetem Luftabscheide-Ventil 6 mittels der Pumpe 4 gefüllt werden. Die Kammer 2 befindet sich auf der Druckseite der Pumpe 4.

Die Förderung der Flüssigkeit aus dem Vorratsbehälter 3 in die erste Kammer 2 erfolgt vorzugsweise mittels des durch die Pumpe 4 erzeugten Unterdrucks. Dies gilt in bevorzugter Ausgestaltung grundsätzlich und nicht nur für das in Figur 15 gezeigte Ausführungsbeispiel.

Beide Kammern weisen Mittel zur Erfassung des Flüssigkeitspegels auf, wie z.B. Ultraschallsensoren, Leitfähigkeitssensoren etc.

Durch die Förderung mittels der Pumpe 4 sinkt der Pegel der Flüssigkeit in der ersten Kammer 1. Unterschreitet dieser ein bestimmtes Niveau, werden beide Kammern mittels des Ventils 7 kurzgeschlossen und über die Ventile 5 und 6 gegenüber der Atmosphäre geöffnet. Dies führt zu einem Anstieg des Flüssigkeitspegels in der Kammer 1 und zu einem Abfall des Flüssigkeitspegels in der Kammer 2.

Bei diesem Vorgang wird die Pumpe 4 entweder verlangsamt, ganz abgeschaltet oder mittels der Kurzschlussleitung bei geöffnetem Ventil 8 fluidmäßig kurzgeschlossen, so dass faktisch keine Förderung in die Kammer 2 erfolgt. Ist die Kammer 1 hinreichend aufgefüllt, wird der Rückführbetrieb durch das Schließen des Ventils 7 und das Umschalten in den normalen Förderbetrieb der Pumpe 4 beendet. Die Pumpe 4 fördern nun wieder Flüssigkeit aus der ersten Kammer 1 in die zweite Kammer 2.

Vorzugsweise befindet sich in der Ablaufleitung der zweiten Kammer 2, die in Figur 15 ganz rechts ohne Bezugszeichen dargestellt ist, ein weiteres Ventil, um die in der Figur 15 dargestellte Entgasungsvorrichtung von dem sich anschließenden Teil einer Vorrichtung, wie z.B. einer hydraulischen Einheit einer Dialysemaschine zu entkoppeln, während das oben beschriebene zweistufige Verfahren (Entgasungsbetrieb - Rückführungsbetrieb) bzw. zumindest der Rückführbetrieb, d.h. der zweite Betriebsmodus läuft.

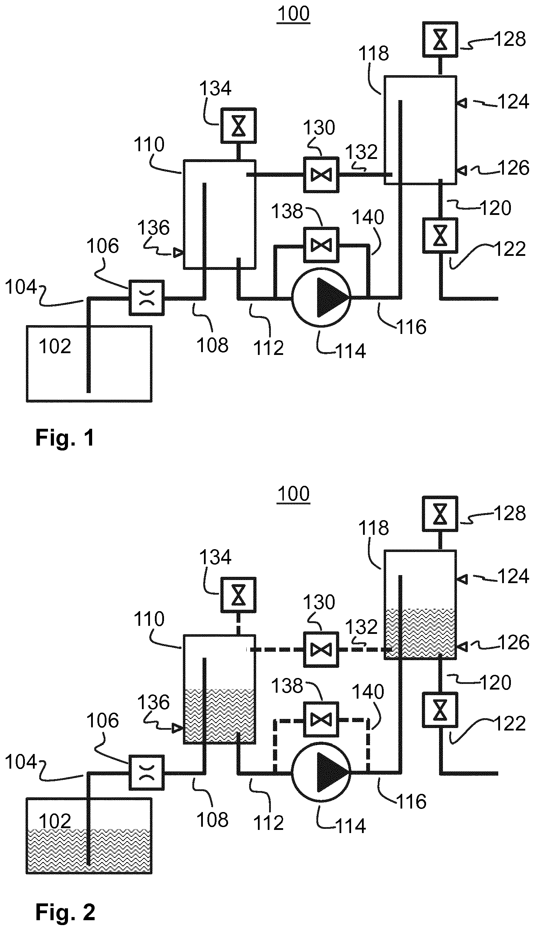

Figur 1 zeigt ein detailliertes Ausführungsbeispiel für eine Vorrichtung 100 zur Entgasung einer Flüssigkeit.

Die Vorrichtung umfasst eine erste Kammer 110 und eine zweite Kammer 118, wobei sich die Kammer 110 hydraulisch vor der Pumpe 114, d.h. auf der Saugseite der Pumpe 114, und die zweite Kammer 118 hydraulisch hinter der Pumpe 114, d.h. auf der Druckseite der Pumpe 114 befindet.

Die Pumpe 114 steht auf ihrer Saugseite mittels der Leitung 112 mit der Kammer 110 und auf ihrer Druckseite mittels der Leitung 116 mit der Kammer 118 in Verbindung. Des Weiteren ist eine Kurzschlussleitung 140 vorgesehen, in der ein Ventil 138 angeordnet ist und die die Druckseite der Pumpe 114 mit deren Saugseite verbindet.

Beide Kammern 110, 118 verfügen jeweils über ein Entlüftungsventil 134, 128, über das die Kammern 110, 118 gegenüber der Atmosphäre geöffnet und somit belüftete werden können.

Wie dies weiter aus Figur 1 hervorgeht, sind die beiden Kammern 110, 118 über eine Rückführleitung 132 direkt miteinander verbunden. In der Leitung 132 befindet sich das Ventil 130.

Die Kammern 110, 118 verfügen darüber hinaus über Levelsensoren 136 (in der ersten Kammer 110) und 124, 126 (in der zweiten Kammer 118). Mittels dieser Levelsensoren lässt sich der Füllstand innerhalb der Kammern bzw. das Über- bzw. Unterschreiten bestimmter Füllstandsgrenzen erfassen. Die Kammer 110 verfügt nur über einen Levelsensor 136, wohingegen die Kammer 118 mit zwei Levelsensoren 124, 126 ausgestattet ist, die in Höhenrichtung voneinander beabstandet sind.

Die erste Kammer steht zulaufseitig mit einem Vorratsbehälter 102 über die Leitung 104, 108 in Verbindung, in der sich eine Drossel 106 befindet.

Die zweite Kammer 118 steht mit einer Ablaufleitung 120, d.h. mit einer Abnahmeeinheit gemäß der Erfindung in Verbindung, in der ein Ventil 122 angeordnet ist. Durch diese Leitung strömt bei geöffnetem Ventil 122 die entgaste Flüssigkeit, die einer Verwendung zugeführt wird und beispielsweise zur Herstellung einer Dialyselösung dient.

Sämtliche der dargestellten Ventile sind je nach Bedarf und Arbeitszyklus geöffnet oder geschlossen.

Bei der Pumpe 114 handelt es sich vorzugsweise um eine nicht okkludierende Pumpe, wie beispielsweise um eine Kreiselpumpe, Impellerpumpe etc.

Das Verfahren zur Luftabscheidung aus der Flüssigkeit wird wie folgt durchgeführt:

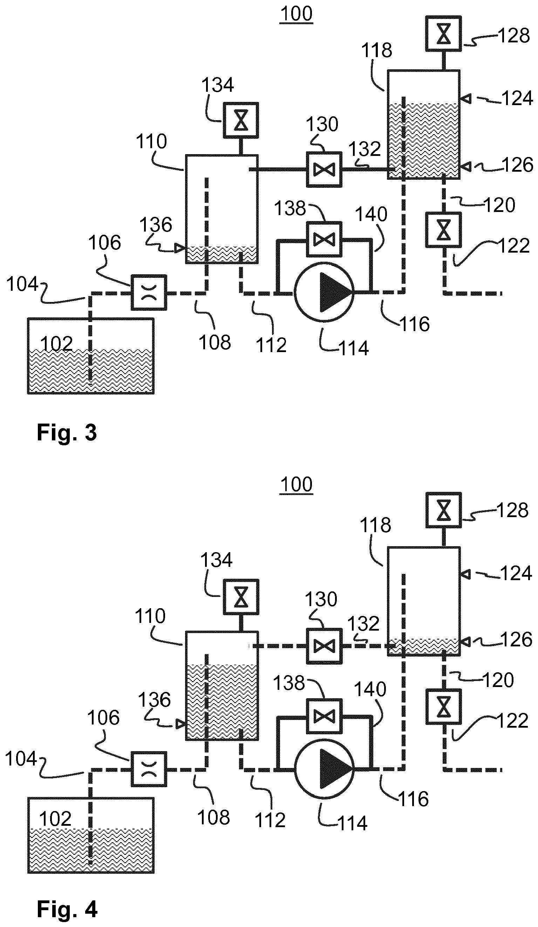

Figur 2 zeigt den Entgasungsbetrieb und Figur 3 den Rückführbetrieb, der sich an den Entgasungsbetrieb anschließt. Die Entgasung erfolgt somit zweistufig, vorzugsweise im Batch-Betrieb, d.h. diskontinuierlich.

Gestrichelte Linien in den Figuren zeigen geschlossene Leitungen, durchgezogene Linien zeigen geöffnete Leitungen.

Der Entgasungsbetrieb gemäß Figur 2 gestaltet sich wie folgt:
Flüssigkeit strömt aus dem Vorratsbehälter 102 durch die Leitungen 104, 106 in die erste Kammer 110 und wird dabei bereits entgast. Der Entgasungsvorgang wird mittels der optional vorhandenen Drossel 106 unterstützt. Das Luft-/Wassergemisch gelangt in die Luftabscheidekammer 110, d.h. in die erste Kammer, deren Entgasungsventil 134 geschlossen ist.

Das Ventil 130 in der Verbindungsleitung, d.h. in der Rückführleitung 132 ist ebenfalls geschlossen. Aus der ersten Kammer 110 fördert die Pumpe 114 die entgaste Flüssigkeit in die Luftabscheidekammer 118, d.h. in die zweite Kammer, deren Entgasungsventil 128 gegenüber der Atmosphäre geöffnet ist, so dass die zweite Kammer 118 belüftet ist. Dies ist vorzugweise dann der Fall, wenn aus der zweiten Kammer 118 aktiv mittels einer nicht dargestellten Pumpe Flüssigkeit entnommen wird oder mittels Schwerkraft. Ist dies nicht der Fall, kann das Ventil 128 geschlossen bleiben, wenn die Pumpe 114 in die zweite Kammer 118 fördert und der sich dann einstellende Überdruck in der zweiten Kammer 118 kann zur Förderung der Flüssigkeit durch die Leitung 120 genutzt werden.

Die Kurzschlussleitung 140 der Pumpe 114 ist durch Schließen des Ventils 138 geschlossen.

Ein Teil der entgasten Flüssigkeit wird über die Leitung 120 einer Verwendung zugeführt, wie beispielsweise eine Vorrichtung zur Zubereitung einer Dialyselösung. Das Ventil 122 der Leitung 120 ist während des Entgasungsbetriebs geöffnet. Ist die Entgasungsleistung, d.h. der Anfall entgaster Flüssigkeit in der zweiten Kammer 118 höher als benötigt, d.h. höher als die Abflussmenge durch die Leitung 120, kann ein oberer Pegelsensor 124 eine Unterbrechung des Entgasungsbetriebs auslösen. Alternativ kann eine Rückführung von Flüssigkeit von der zweiten Kammer 118 im laufenden Betrieb, d.h. bei laufender Pumpe 114 in die erste Kammer 110 erfolgen; in diesem Fall wird die Leitung 132 geöffnet. Dieses Prinzip findet nur dann Anwendung bzw. funktioniert nur dann, wenn aktiv mittels einer Pumpe aus der zweiten Kammer 118 gefördert wird oder die Schwerkraft für den Abfluss mittels der Leitung 120 ausreicht und wenn die Zufuhr entgaster Flüssigkeit höher ist als die Nettoentnahme aus der zweiten Kammer 118.

Der Entgasungsbetrieb gemäß Figur 2 wird beendet, wenn der Pegelsensor 136 der ersten Kammer 110 ausgelöst hat, d.h. wenn das Niveau der Flüssigkeit in der ersten Kammer 110 bis zu dem Pegelsensor 136 gefallen ist.

Figur 3 zeigt den sich dann anschließenden Rückführbetrieb, d.h. den zweiten Betriebsmodus. Die Pumpe 114 wird kurzgeschlossen, indem die Leitung 140 durch Öffnen des Ventils 138 geöffnet wird. Es findet somit keine oder im Wesentlichen keine Nettoförderung von der ersten in die zweite Kammer statt. Die Leitungen 112 und 116 werden somit nicht oder nur unwesentlich durchströmt. Somit fließt auch in den Leitungen 104 und 108 keine oder im Wesentlichen keine Flüssigkeit, wie dies durch die gestrichelte Linie gekennzeichnet ist, da die Pumpe in der ersten Kammer 110 keinen Unterdruck erzeugt, der gemäß Figur 2 zu einer Einströmung von Flüssigkeit aus dem Vorratsbehälter 102 in die erste Kammer 110 führt.

Die Leitung 120 ist mittels des Ventils 122 geschlossen, d.h. es erfolgt keine Entnahme entgaster Flüssigkeit an einen Abnehmer. Die Leitung 132 bzw. deren Ventil 130 ist geöffnet, so dass entgaste Flüssigkeit von der zweiten in die erste Kammer zurückfließt. Die Entlüfter 128 und 134 sind dabei geöffnet.

Die Rückführung von der zweiten in die erste Kammer erfolgt durch Schwerkraft. Grundsätzlich ist von der Erfindung auch der Fall umfasst, dass dazu eine Pumpe verwendet wird.

Durch diese Rückführung sinkt der Flüssigkeitspegel in der zweiten Kammer 118 und er steigt in der ersten Kammer 110. Das Ende des Rückführbetriebes ist in Figur 4 gezeigt und ist dadurch gekennzeichnet, dass der Flüssigkeitspegel in der zweiten Kammer 118 den unteren Pegelsensor 126 erreicht.

Im Anschluss daran werden alle Leitungen sowie die Pumpe 114 wieder in den Entgasungsbetrieb, d.h. in den ersten Betriebsmodus geschaltet, wie er in Figur 2 gezeigt ist.

Die Vorrichtung gemäß der Figuren 1 - 4 arbeitet vorzugsweise diskontinuierlich ("batch-degassing"), d.h. es wird nicht kontinuierlich, sondern nur batchweise entgaste Flüssigkeit zum Abzug durch die Leitung 120 bereitgestellt.

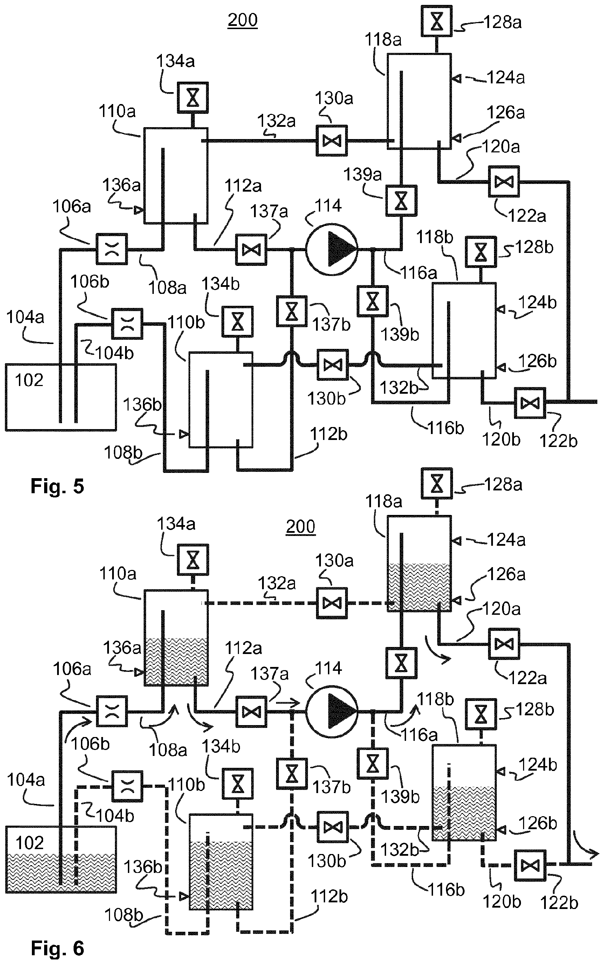

Von der Erfindung ist jedoch auch die kontinuierliche Bereitstellung entgaster Flüssigkeit umfasst. Hierzu wird auf die Figuren 5 bis 8 verwiesen.

Dazu werden zwei identische Entgasungssysteme, wie sie zu den Figuren 1 bis 4 beschrieben wurden parallel vorgesehen, wobei gleiche Bezugszeichen in den Figuren 5 bis 8 gleiche oder funktionsgleiche Teile bezeichnen, wie in den Figuren 1 bis 4 und wobei die Teile des einen Entgasungssystems (im Folgenden als "Entgasungssystem a" bezeichnet) mit dem ergänzenden Buchstaben a und die die Teile des anderen Entgasungssystems (im Folgenden als "Entgasungssystem b" bezeichnet) mit dem ergänzenden Buchstaben b gekennzeichnet sind. Das Gesamtsystem ist mit 200 bezeichnet.

Wie dies aus Figur 5 hervorgeht, stehen beide erste Kammern 110a und 110b jeweils mit demselben Vorratsbehälter 102 in Verbindung und beziehen aus diesem die zu entgasende Flüssigkeit. Des Weiteren münden die Ablaufleitungen der beiden zweiten Kammern 118a und 118b in dieselbe Ablaufleitung, in der die entgaste Flüssigkeit zur weiteren Verwendung bereitgestellt wird.

Zum Umschalten zwischen beiden Entgasungssystemen a und b dienen die Ventile 137a und 137b, die jeweils zwischen den ersten Kammern 110a und 110b und der gemeinsamen Pumpe 114 angeordnet sind und die Ventile 139a und 139b, die jeweils zwischen der gemeinsamen Pumpe 114 und den zweiten Kammern 118a und 118b angeordnet sind.

Die beiden Entgasungssysteme a und b werden im Wechsel betrieben, d.h. erfolgt in einem Entgasungssystem die Entgasung (erster Betriebsmodus), erfolgt im anderen System die Rückführung (zweiter Betriebsmodus) und umgekehrt, so dass stets bei abwechselnd geöffneten Ventilen 122a oder 122b entgaste Flüssigkeit in der Leitung zur weiteren Verwendung abgezogen werden kann.

Figur 6 zeigt den Zustand, in dem im Entgasungssystem a die Entgasung erfolgt und über die Leitung 120 a bei geöffnetem Ventil 122a entgaste Flüssigkeit bereitgestellt wird. Dazu fördert die Pumpe 114 bei geöffnetem Ventil 137a und geschlossenem Ventil 137 b Flüssigkeit aus der ersten Kammer 110a in die zweite Kammer 118 a. In dem Entgasungssystem b sind sämtliche Ventile geschlossen, so dass dort weder eine Entgasung noch eine Rückführung von der zweiten in die erste Kammer stattfindet.

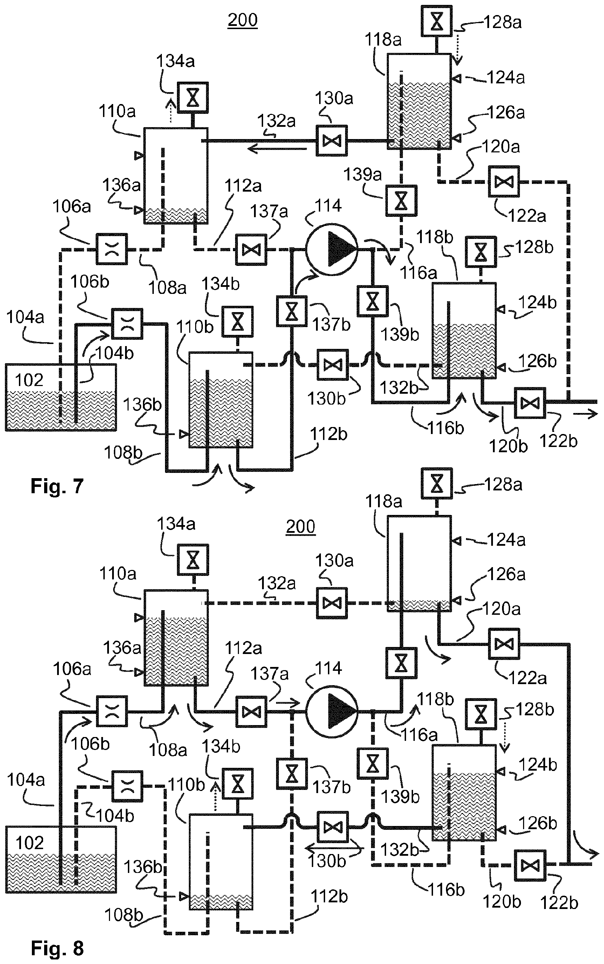

Sobald der Pegel der Flüssigkeit in der ersten Kammer 110a den Pegelsensor 136a erreicht hat, beginnt in dem Entgasungssystem a die Rückführung durch Öffnen der Rückführleitung 132a und in dem Entgasungssystem b die Entgasung durch Öffnen der Ventile 137b, 139b und 122b. Dies ist in Figur 7 gezeigt. Das Ventil 122a ist geschlossen, so dass entgaste Flüssigkeit nur aus der zweiten Kammer 118b bereit gestellt wird.

Hat der Flüssigkeitspegel in der ersten Kammer 110b des Entgasungssystems b den Pegelsensor 136b erreicht, wie dies in Figur 8 gezeigt ist, wird auf den in Figur 8 gezeigten Zustand umgeschaltet, in dem in dem Entgasungssystem a die Entgasung und durch Öffnen des Ventils 122a Bereitstellung entgaster Flüssigkeit erfolgt und in dem in dem Entgasungssystem b bei geschlossenem Ventil 122b und geöffnetem Ventil 130b die Rückführung von der zweiten Kammer 118b in die erste Kammer 110b erfolgt. Des Weiteren werden die Ventile 137a und 139a geöffnet und die Ventile 137b und 139b geschlossen.

Hat der Füllstand in der ersten Kammer 110a des Entgasungssystems a den Pegelsensor 136a erreicht, wird wieder in den Zustand gemäß Figur 7 umgeschaltet.

Aufgrund des Wechsels des Betriebs der parallel angeordneten Entgasungssysteme a, b kann an dem Ablauf, d.h. an der Abnahmeeinheit stromabwärts der Ventile 122a/122b stets entgaste Flüssigkeit bereit gestellt werden.

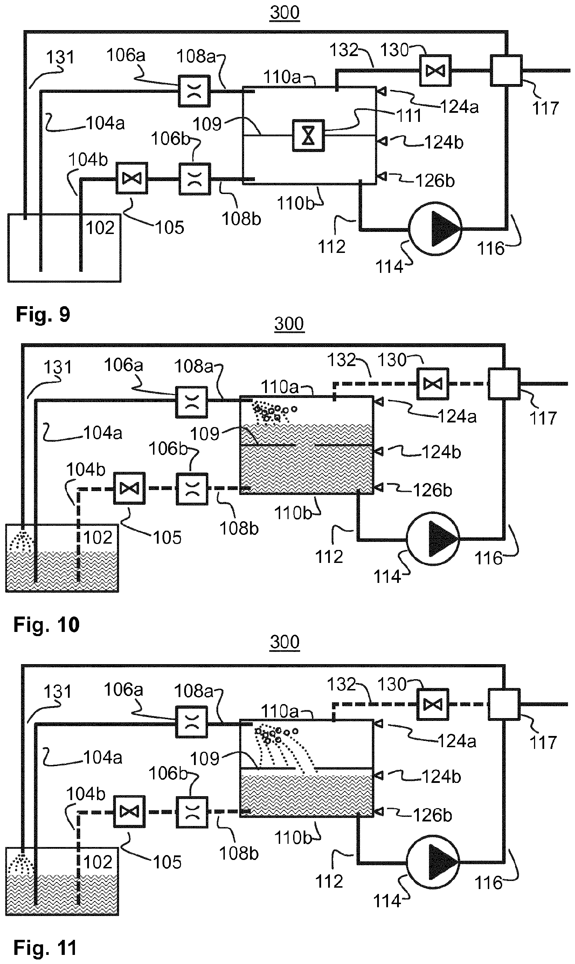

Die Figuren 9 bis 14 zeigen eine weitere Ausgestaltung eines Entgasungssystems 300 gemäß der Erfindung.

Figur 9 zeigt den Aufbau des Systems mit einer ersten 110a und einer zweiten Entgasungskammer 11 0b, die durch eine Trennwand 109 voneinander getrennt sind, in der ein Ventil 111 angeordnet ist. Wie dies aus Figur 9 hervorgeht, befindet sich die zweite Kammer oberhalb der ersten Kammer.

Beide Kammern 110a, 110b stehen über Leitungen 104a, 104b, 108a, 108b in denen vorzugsweise je eine Drossel 106a, 106b angeordnet ist, mit dem Vorratsbehälter 102 in Verbindung. In der zu der zweiten Kammer 110b führenden Leitung ist zudem ein Ventil 105 angeordnet. Die Pumpe steht mit einer Saugleitung 112 in Verbindung, die in die zweite Kammer 110b mündet, sowie mit einer Druckleitung 116, die zu einem Abzweig 117, d.h. zu der Abnahmeeinheit führt. Von diesem Abzweig führt ein nach rechts weisender Anschluss zur weiteren Verwendung der entgasten Flüssigkeit. Dieser Anschluss kann beispielsweise mit einer Vorrichtung zur Herstellung von Dialyselösung oder mit einem Dialysegerät verbunden sein.

Eine weitere Leitung führt von dem Abzweig 117 zurück zu dem Vorratsbehälter (Leitung 131) und eine weitere Leitung führt von dem Abzweig 117 zurück zu der ersten Kammer 110a (Leitung 132 mit Ventil 130).

Das Bezugszeichen 124a kennzeichnet einen Pegelsensor in der ersten Kammer 110a und die Bezugszeichen 124b und 126b kennzeichnen Pegelsensoren in der zweiten Kammer 110b.

In dem Zustand gemäß Figur 10 (erste Phase der Entgasung - erster Betriebsmodus) fördert die Pumpe 114 bei geschlossenem Ventil 105 über die Leitung 104a und die Drossel 106a sowie die Leitung 108a in die erste Entgasungskammer 110a. Dazu dient der durch die Pumpe 114 erzeugte Unterdruck in den fluidmäßig verbundenen Entgasungskammern. Das durch Kreise dargestellte Gas sowie Flüssigkeit (Punkte) treten separiert über die Leitung 108a in die erste Entgasungskammer 110a ein.

Die Pumpe fördert die entgaste Flüssigkeit aus der zweiten Entgasungskammer 110b zu der Abzweigung 117. Ein Teil der Flüssigkeit wird für eine Verwendung, wie z.B. zur Herstellung einer Dialyselösung herangezogen, ein anderer Teil der Flüssigkeit wird über die Rückführleitung 131 in den Vorratsbehälter 102 zurück gefördert.

Die Rückführleitung 132 ist mittels des Ventils 130 abgesperrt. Der Ansaugzweig 104b, 108b zu der zweiten Entgasungskammer 110b ist mittels des Ventils 105 abgesperrt.

Sinkt der Pegel in den durch das geöffnete Ventil 111 verbundenen Entgasungskammern unter das Niveau des Sensors 124b, wie dies in Figur 11 dargestellt ist, wird die zweite Phase des Entgasungsbetriebs und somit auch der Rückführbetrieb (zweiter Betriebsmodus) initiiert.

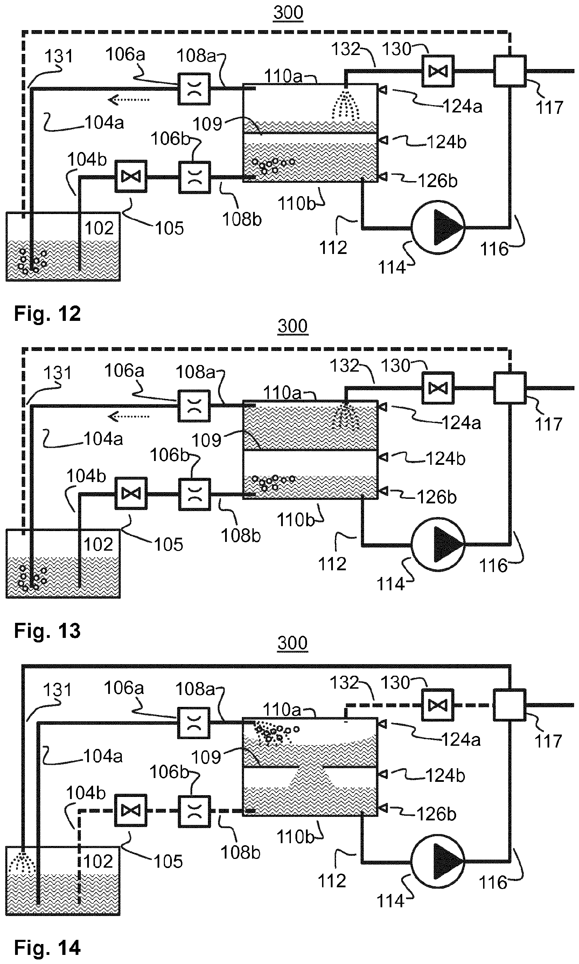

Diese zweite Phase ist in Figur 12 wiedergegeben. Das Ventil 11 ist geschlossen und trennt somit die beiden Entgasungskammern fluidmäßig voneinander. Die Rückführungleitung 132 wird durch Öffnen des Ventils 130 geöffnet und der Ansaugzweig 104b, 108b von dem Vorratsbehälter 102 zu der zweiten Entgasungskammer 110b wird durch Öffnen des Ventils 105 geöffnet.

Die Pumpe 114 fördert Flüssigkeit aus der zweiten Entgasungskammer 110b und Flüssigkeit wird aus dem Vorratsbehälter über die Leitung 104b, 108b in die zweite Entgasungskammer 110b angesaugt und entgast. Durch die Förderung der Pumpe 114 wird die zweite Entgasungskammer 110b allmählich entleert und der zuvor über die Rückführleitung 131 in den Vorratsbehälter rückgeführte Teil der entgasten Flüssigkeit wird nun über die geöffnete Rückführleitung 132 in die erste Entgasungskammer 110a zurückgeführt. Verdrängte Luft entweicht über den Ansaugzweig 104a und 108a entsprechend des gestrichelten Pfeils und ist in dem Vorratsbehälter 102 in Form von Blasen dargestellt. Alternativ oder zusätzlich kommt ein nicht dargestelltes Entgasungsventil zum Einsatz.

Löst der Pegelsensor 124a der ersten Entgasungskammer 110a aus, weil diese nun entsprechend bzw. weitgehend gefüllt ist, endet die zweite Phase des Entgasungsbetriebs. In der Zwischenzeit hat sich die zweite Entgasungskammer 110b durch den Betrieb der Pumpe 114 weiter entleert.

Das Auslösen des Pegelsensors 124a führt dazu, dass das Ventil 111 wieder geöffnet wird, (vgl. Figur 14) so dass die beiden Entgasungskammern wieder miteinander verbunden sind. Entgaste Flüssigkeit fließt nun von der ersten Kammer 110a durch das Ventil 111 in die zweite Kammer 110b und füllt diese auf. Die Rückführleitung 132 ist durch Schließen des Ventils 130 abgesperrt und ein nicht zur Verwendung abgeführter Teil der Flüssigkeit strömt über die Leitung 131 zurück in den Vorratsbehälter 102. Wie in dem Zustand gemäß Figur 10 ist der Ansaugzweig 104b und 108b zu der zweiten Kammer abgesperrt und der Unterdruck in den miteinander verbundenen Kammern saugt Flüssigkeit aus dem Vorratsbehälter 102 über den Ansaugzweig 104a und 108a in die erste Kammer 110a.

Sowohl in dem Betriebsmodus gemäß Figur 10 als auch in dem Betriebsmodus gemäß Figur 13 wird an der Abzweigung 117 entgaste Flüssigkeit bereit gestellt, so dass ein kontinuierlicher Betrieb oder quasi kontinuierlicher Betrieb vorliegt.

## Patentansprüche

1. Vorrichtung (100) zur Entgasung von Flüssigkeiten umfassend ein Entgasungssystem, wobei das Entgasungssystem eine erste Entgasungskammer (110a), eine zweite Entgasungskammer (110b), einen Flüssigkeitsvorrat (102), eine Pumpe (114) sowie eine Zufuhrleitung aufweist, die den Flüssigkeitsvorrat (102) mit der ersten Entgasungskammer (110a) verbindet, wobei die Pumpe (114) saugseitig mit der ersten Entgasungskammer (110a) und druckseitig mit der zweiten Entgasungskammer (110b) verbunden ist, wobei das Entgasungssystem des Weiteren eine absperrbare Rückführungsleitung (132) aufweist, die die beiden Entgasungskammern (110a, 110b) miteinander verbindet, und wobei die Vorrichtung einen Kontroller umfasst, der ausgebildet ist, das Entgasungssystem in einem ersten Betriebsmodus und in einem zweiten Betriebsmodus zu betreiben, wobei in dem ersten Betriebsmodus das Entgasungssystem derart geschaltet ist, dass die Pumpe (114) Flüssigkeit aus der ersten Entgasungskammer (110a) abführt und einer Abnahmeeinheit für entgaste Flüssigkeit zuführt, **dadurch gekennzeichnet, dass** das Entgasungssystem in dem zweiten Betriebsmodus derart geschaltet ist, dass Flüssigkeit durch die Rückführungsleitung (132) aus der zweiten (110b) in die erste Entgasungskammer (110a) rückgeführt wird.

2. Vorrichtung (100) nach Anspruch 1, **dadurch gekennzeichnet, dass** die Pumpe (114) saugseitig unmittelbar oder mittelbar mit der ersten Entgasungskammer (110a) und druckseitig unmittelbar oder mittelbar mit der zweiten Entgasungskammer (110b) bzw. mit der Abnahmeeinheit verbunden ist.

3. Vorrichtung (100) nach Anspruch 1 oder 2, **dadurch gekennzeichnet, dass** der Kontroller derart ausgebildet ist, dass die Pumpe (114) in beiden Betriebsmodi in einem Fördermodus betrieben wird oder dass die Pumpe (114) nur in dem ersten Betriebsmodus, nicht aber in dem zweiten Betriebsmodus in einem Fördermodus betrieben wird.

4. Vorrichtung (100) nach einem der vorhergehenden Ansprüche, **dadurch gekennzeichnet, dass** die erste (110a) und die zweite (110b) Entgasungskammer derart relativ zueinander angeordnet sind, dass die Flüssigkeit schwerkraftbedingt oder bedingt durch die Förderkraft der Pumpe (114) durch die Rückführungsleitung (132) aus der zweiten (110b) in die erste (110a) Entgasungskammer gefördert wird.

5. Vorrichtung (100) nach einem der vorhergehenden Ansprüche, **dadurch gekennzeichnet**, das die beiden Entgasungskammern (110a, 110b) räumlich voneinander getrennt sind oder in einer gemeinsamen baulichen Einheit angeordnet sind, die eine Trennwand (109) aufweist, die die beiden Entgasungskammern (110a, 100b) voneinander trennt.

6. Vorrichtung (100) nach einem der vorhergehenden Ansprüche, **dadurch gekennzeichnet, dass** nur die erste Entgasungskammer (110a) mit dem Flüssigkeitsvorrat (102) verbunden ist oder dass beide Entgasungskammern (110a, 110b) mit dem Flüssigkeitsvorrat (102) verbunden sind.

7. Vorrichtung (100) nach einem der vorhergehenden Ansprüche, **dadurch gekennzeichnet, dass** zwei parallel geschaltete Entgasungssysteme vorgesehen sind und dass der Kontroller derart ausgebildet ist, dass die Entgasungssysteme abwechselnd derart betrieben werden, dass das erste Entgasungssystem in dem ersten Betriebsmodus und das zweite Entgasungssystem zeitgleich in dem zweiten Betriebsmodus betrieben wird und umgekehrt.

8. Vorrichtung (100) nach einem der vorhergehenden Ansprüche, **dadurch gekennzeichnet, dass** in dem ersten (110a) und/oder in dem zweiten (110b) Entgasungsbehälter ein Levelsensor (136) zur Erfassung des Flüssigkeitsniveaus vorhanden ist und dass der Kontroller ausgebildet ist, von dem ersten auf den zweiten Betriebsmodus oder von dem zweiten auf den ersten Betriebsmodus umzuschalten, wenn ein Levelsensor (136) auslöst.

9. Verfahren zum Entgasen von Flüssigkeiten mittels einer Vorrichtung nach einem der Ansprüche 1 bis 8, wobei in dem ersten Betriebsmodus Flüssigkeit aus der ersten Entgasungskammer (110a) abgezogen und an einer Abnahmeeinheit für entgaste Flüssigkeit zur Verfügung gestellt wird, **dadurch gekennzeichnet, dass** in dem zweiten Betriebsmodus Flüssigkeit von der zweiten Entgasungskammer (110b) in die erste Entgasungskammer (110a) zurückgeführt wird.

10. Verfahren nach Anspruch 9, **dadurch gekennzeichnet, dass** in dem ersten Betriebsmodus Flüssigkeit aus der ersten Entgasungskammer (110a) über die zweite Entgasungskammer (110b) oder unter Ausschluss der zweiten Entgasungskammer (110b) mittels der Pumpe (114) zu der Abnahmeeinheit gefördert wird.

11. Verfahren nach Anspruch 9 oder 10, **dadurch gekennzeichnet, dass** von dem ersten auf den zweiten Betriebsmodus umgeschaltet wird, wenn das Flüssigkeitsniveau in der ersten Entgasungskammer (110a) einen Grenzwert unterschreitet und/oder in der zweiten Entgasungskammer (110b) einen Grenzwert überschreitet.

12. Verfahren nach einem der Ansprüche 9 bis 11, **dadurch gekennzeichnet, dass** von dem zweiten auf den ersten Betriebsmodus umgeschaltet wird, wenn das Flüssigkeitsniveau in der zweiten Entgasungskammer (110b) einen Grenzwert unterschreitet und/oder in der ersten Entgasungskammer (110a) einen Grenzwert überschreitet.

13. Verfahren nach einem der Ansprüche 9 bis 12, **dadurch gekennzeichnet, dass** entgaste Flüssigkeit nur in dem ersten Betriebsmodus oder sowohl in dem ersten sowie auch in dem zweiten Betriebsmodus an der Abnahmeeinheit bereitgestellt wird.

14. Verfahren nach einem der Ansprüche 9 bis 13, **dadurch gekennzeichnet, dass** beide Entgasungskammern (110a, 110b) in einer gemeinsamen baulichen Einheit angeordnet sind, die eine Trennwand (109) aufweist, die die beiden Entgasungskammern (110a, 110b) voneinander trennt, wobei in der Trennwand (109) ein Ventil (111) oder ein sonstiges Absperrelement vorgesehen ist, das in dem ersten Betriebsmodus geöffnet und in dem zweiten Betriebsmodus geschlossen ist.

15. Verwendung einer Vorrichtung gemäß einem der Ansprüche 1 bis 8 oder eines Verfahrens gemäß einem der Ansprüche 9 bis 14 zur Herstellung einer medizinischen Lösung, insbesondere zur Herstellung einer Dialyselösung.

## Claims

1. An apparatus (100) for degassing liquids comprising a degassing system, wherein the degassing system has a first degassing chamber (110a), a second degassing chamber (110b), a liquid store (102), a pump (114), and a supply line that connects the liquid store (102) to the first degassing chamber (110a), wherein the pump (114) is connected to the first degassing chamber (110a) at the intake side and to the second degassing chamber (11 0b) at the pressure side, wherein the degassing system furthermore has a return line (132) that can be cut off and that connects the two degassing chambers (110a, 110b) to one another, and wherein the apparatus comprises a controller that is configured to operate the degassing system in a first operating mode and in a second operating mode, wherein the degassing system is connected in the first operating mode such that the pump (114) conducts liquid away from the first degassing chamber (110a) and supplies it to a removal unit for degassed liquid, **characterized in that** the degassing system is connected in the second operating mode such that liquid is returned from the second (110b) to the first degassing chamber (110a) through the return line (132).

2. An apparatus (100) in accordance with claim 1, **characterized in that** the pump (114) is connected directly or indirectly to the first degassing chamber (110a) at the intake side and is connected directly or indirectly to the second degassing chamber (110b) or to the removal unit at the pressure side.

3. An apparatus (100) in accordance with claim 1 or claim 2, **characterized in that** the controller is configured such that the pump (114) is operated in a conveying mode in both operating modes or **in that** the pump (114) is only operated in a conveying mode in the first operating mode, but not in the second operating mode.

4. An apparatus (100) in accordance with one of the preceding claims, **characterized in that** the first (110a) and the second (110b) degassing chamber are arranged relative to one another such that the liquid is conveyed through the return line (132) from the second (110b) into the first (110a) degassing chamber due to gravity or due to the conveying power of the pump (114).

5. An apparatus (100) in accordance with one of the preceding claims, **characterized in that** the two degassing chambers (110a, 110b) are spatially separated from one another or are arranged in a common construction unit that has a partition wall (109) that separates the two degassing chambers (110a, 100b) from one another.

6. An apparatus (100) in accordance with one of the preceding claims, **characterized in that** only the first degassing chamber (110a) is connected to the liquid store (102) or **in that** both degassing chambers (110a, 110b) are connected to the liquid store (102).

7. An apparatus (100) in accordance with one of the preceding claims, **characterized in that** two degassing systems are provided that are connected in parallel, and **in that** the controller is configured such that the degassing systems are operated alternately such that the first degassing system is operated in the first operating mode and the second degassing system is simultaneously operated in the second operating mode and vice versa.

8. An apparatus (100) in accordance with one of the preceding claims, **characterized in that** a level sensor (136) for detecting the liquid level is present in the first (110a) and/or in the second (110b) degassing container, and **in that** the controller is configured to switch over from the first operating mode to the second operating mode or from the second operating mode to the first operating mode when a level sensor (136) fires.

9. A method for degassing liquids by means of an apparatus in accordance with one of the claims 1 to 8, wherein liquid is withdrawn from the first degassing chamber (110a) and is provided to a removal unit for degassed liquid in the first operating mode, **characterized in that** liquid is led back from the second degassing chamber (110b) into the first degassing chamber (110a) in the second operating mode.

10. A method in accordance with claim 9, **characterized in that** liquid is conveyed from the first degassing chamber (110a) via the second degassing chamber (110b) or to the exclusion of the second degassing chamber (110b) to the removal unit by means of the pump (114) in the first operating mode.

11. A method in accordance with claim 9 or claim 10, **characterized in that** a switchover is made from the first operating mode to the second operating mode when the liquid level in the first degassing chamber (110a) falls below a limit value and/or exceeds a limit value in the second degassing chamber (110b).

12. A method in accordance with one of the claims 9 to 11, **characterized in that** a switchover is made from the second operating mode to the first operating mode when the liquid level in the second degassing chamber (110b) falls below a limit value and/or exceeds a limit value in the first degassing chamber (110a).

13. A method in accordance with one of the claims 9 to 12, **characterized in that** degassed liquid is only provided to the removal unit in the first operating mode or both in the first operating mode and in the second operating mode.

14. A method in accordance with one of the claims 9 to 13, **characterized in that** both degassing chambers (110a, 110b) are arranged in a common construction unit that has a partition wall (109) that separates the two degassing chambers (110a, 110b) from one another, wherein a valve (111) or another cut-off element is provided in the partition wall (109) that is opened in the first operating mode and is closed in the second operating mode.

15. Use of an apparatus in accordance with one of the claims 1 to 8 or of a method in accordance with one of the claims 9 to 14 for preparing a medical solution, in particular for preparing a dialysis solution.

## Revendications

1. Dispositif (100) pour dégazer des liquides, comprenant un système de dégazage, le système de dégazage présentant une première chambre de dégazage (110a), une seconde chambre de dégazage (110b), une réserve de liquide (102), une pompe (114) ainsi qu'une conduite d'alimentation qui relie la réserve de liquide (102) à la première chambre de dégazage (110a), la pompe (114) étant reliée côté aspiration à la première chambre de dégazage (110a) et côté refoulement à la seconde chambre de dégazage (110b), le système de dégazage présentant en outre une conduite de retour (132) pouvant être fermée, laquelle relie les deux chambres de dégazage (110a, 110b) entre elles, et le dispositif comprenant un contrôleur, qui est conçu pour exploiter le système de dégazage dans un premier mode de fonctionnement et dans un second mode de fonctionnement, le système de dégazage étant, dans le premier mode de fonctionnement, commuté de telle manière que la pompe (114) prélève du liquide de la première chambre de dégazage (110a) et l'alimente dans une unité de réception pour liquide dégazé, **caractérisé en ce que**, dans le second mode de fonctionnement, le système de dégazage est commuté de telle manière que du liquide est renvoyé de la seconde (110b) à la première chambre de dégazage (110a) par la conduite de retour (132).

2. Dispositif (100) selon la revendication 1, **caractérisé en ce que** la pompe (114) est reliée, côté aspiration, directement ou indirectement à la première chambre de dégazage (110a) et, côté refoulement, directement ou indirectement à la seconde chambre de dégazage (110b) ou à l'unité de réception.

3. Dispositif (100) selon la revendication 1 ou 2, **caractérisé en ce que** le contrôleur est conçu de telle manière que, dans les deux modes de fonctionnement, la pompe (114) est exploitée dans un mode de refoulement ou **en ce que** la pompe (114) est exploitée dans un mode de refoulement uniquement dans le premier mode de fonctionnement mais pas dans le second mode de fonctionnement.

4. Dispositif (100) selon l'une des revendications précédentes, **caractérisé en ce que** la première (110a) et la seconde (110b) chambre de dégazage sont disposées l'une par rapport à l'autre de telle manière que le liquide est refoulé de la seconde (110b) à la première (110a) chambre de dégazage à travers la conduite de retour (132) par la force de gravité ou par la force de refoulement de la pompe (114).

5. Dispositif (100) selon l'une des revendications précédentes, **caractérisé en ce que** les deux chambres de dégazage (110a, 110b) sont séparées l'une de l'autre dans l'espace ou sont disposées dans une unité structurale commune qui présente une paroi de séparation (109) qui sépare les deux chambres de dégazage (110a, 100b) l'une de l'autre.

6. Dispositif (100) selon l'une des revendications précédentes, **caractérisé en ce que** seule la première chambre de dégazage (110a) est reliée à la réserve de liquide (102) ou **en ce que** les deux chambres de dégazage (110a, 110b) sont reliées à la réserve de liquide (102).

7. Dispositif (100) selon l'une des revendications précédentes, **caractérisé en ce que** deux systèmes de dégazage couplés en parallèle sont prévus et **en ce que** le contrôleur est conçu de telle manière que les systèmes de dégazage sont exploités alternativement de telle manière que le premier système de dégazage est exploité dans le premier mode de fonctionnement et en même temps le second système de dégazage dans le second mode de fonctionnement et inversement.

8. Dispositif (100) selon l'une des revendications précédentes, **caractérisé en ce qu'**un capteur de niveau (136) se trouve dans le premier (110a) et/ou dans le second (110b) récipient de dégazage pour détecter le niveau de liquide et **en ce que** le contrôleur est conçu pour commuter du premier au second mode de fonctionnement ou du second au premier mode de fonctionnement quand un capteur de niveau (136) se déclenche.

9. Procédé pour dégazer des liquides au moyen d'un dispositif selon l'une des revendications 1 à 8, dans lequel, dans le premier mode de fonctionnement, du liquide est retiré de la première chambre de dégazage (110a) et mis à disposition d'une unité de réception pour liquide dégazé, **caractérisé en ce que**, dans le second mode de fonctionnement, du liquide est renvoyé de la seconde chambre de dégazage (110b) à la première chambre de dégazage (110a).

10. Procédé selon la revendication 9, **caractérisé en ce que**, dans le premier mode de fonctionnement, du liquide est refoulé de la première chambre de dégazage (110a) à l'unité de réception au moyen de la pompe (114) par le biais de la seconde chambre de dégazage (110b) ou sans passer par la seconde chambre de dégazage (110b).

11. Procédé selon la revendication 9 ou 10, **caractérisé en ce que** la commutation du premier au second mode de fonctionnement a lieu quand le niveau de liquide devient inférieur à une valeur limite dans la première chambre de dégazage (110a) et/ou dépasse une valeur limite dans la seconde chambre de dégazage (110b).

12. Procédé selon l'une des revendications 9 à 11, **caractérisé en ce que** la commutation du second au premier mode de fonctionnement a lieu quand le niveau de liquide devient inférieur à une valeur limite dans la seconde chambre de dégazage (110b) et/ou dépasse une valeur limite dans la première chambre de dégazage (110a).

13. Procédé selon l'une des revendications 9 à 12, **caractérisé en ce que** du liquide dégazé est mis à disposition de l'unité de réception uniquement dans le premier mode de fonctionnement ou aussi bien dans le premier que dans le second mode de fonctionnement.

14. Procédé selon l'une des revendications 9 à 13, **caractérisé en ce que** les deux chambres de dégazage (110a, 110b) sont disposées dans une unité structurale commune, qui présente une paroi de séparation (109) qui sépare les deux chambres de dégazage (110a, 110b) l'une de l'autre, une vanne (111) ou un autre élément de blocage étant prévu dans la paroi de séparation (109), ladite vanne étant ouverte dans le premier mode de fonctionnement et fermée dans le second mode de fonctionnement.

15. Utilisation d'un dispositif selon l'une des revendications 1 à 8 ou d'un procédé selon l'une des revendications 9 à 14 pour produire une solution médicale, en particulier pour produire une solution de dialyse.
